# EUROPEAN PATENT SPECIFICATION

(11) **EP 3 512 362 B1**
(45) Date of publication and mention of the grant of the patent: **14.04.2021**
(21) Application number: 17758207.9
(22) Date of filing: 31.08.2017
(51) Int. Cl.: A24F 40/46

(54) **ELECTRONIC AEROSOL-GENERATING SMOKING DEVICE**
ELEKTRONISCHE AEROSOLERZEUGENDE RAUCHVORRICHTUNG
DISPOSITIF À FUMER ÉLECTRONIQUE À GÉNÉRATION D'AÉROSOL

(30) Priority: 15.09.2016 EP 16189009
(43) Date of publication of application: 24.07.2019
(73) Proprietor: Philip Morris Products S.A., 2000 Neuchâtel (CH)
(72) Inventor: BESSANT, Michel, 2000 Neuchâtel (CH); DUC, Fabien, 1227 Carouge (CH)
(74) Representative: Sánchez García, Juan
(86) International application number: PCT/EP2017/071928
(87) International publication number: WO 2018/050449

(56) References cited:
- EP-A1- 2 460 422
- WO-A1-2014/102092
- WO-A2-2013/076098
- WO-A2-2015/177304
- DE-U1- 20 114 878

## Description

The invention refers to electronic aerosol-generating smoking devices.

Various smoking systems are known, wherein an aerosol-forming substrate is heated in order to vaporize substances from the substrate forming an inhalable aerosol. In some systems a tobacco material plug is heated by a heater blade inserted into the plug. Residues left on the blade as well as deposits in a flow path may influence a smoking experience. In addition, cleaning of device parts is often not possible or only with high effort.

WO 2015/177304 discloses an electrically heated aerosol-generating system comprising a tubular housing in turn having an opening at one end for receiving an aerosol-forming article. At the opposite end of the tubular housing is a substantially cylindrical heater element, a rechargeable battery for powering the heater element, and control electronics for controlling the operation of the system. The control electronics control the switching on and off of the system, the power supplied from the rechargeable battery to the heater element and the charging of the rechargeable battery when the battery is connected to an external power supply.

WO 2013/076098 discloses an aerosol-generating device including an extractor. The extractor comprises a sleeve and a sliding receptacle in the form of holder. The holder comprises a substantially cylindrical tube, but has a diameter slightly smaller than that of the sleeve, such that holder can be slidably received in sleeve. The outer end of the holder is open to receive an aerosol-forming substrate and includes a flange. The flange can be in the form of a projecting rim or collar, which abuts against the outer end of sleeve when the extractor is in a first, operating position.

There is need for aerosol-generating devices reducing the amount of residues, in particular reducing the requirement of cleaning device parts subject to aerosol-forming substrate or components thereof.

According to the invention, there is provided an electronic aerosol-generating smoking device according to claim 1. The electronic smoking device comprises a main body comprising a heating region for aerosol-forming substrate, wherein at least portions of internal surfaces of the main body in the heating region or downstream of the heating region are a hydrophobic or super-hydrophobic surface.

An electronic aerosol-generating smoking device according to the invention comprises a main body comprising a heating region for aerosol-forming substrate, wherein the main body comprises: a cavity for receiving an aerosol-forming substrate; and a heater arranged in the main body and adapted for heating aerosol-forming substrate accommodated in the cavity. At least a portion of an internal surface of the main body defining the cavity in the heating region is a hydrophobic or super-hydrophobic surface. The heating region comprises a tubular-shaped extractor, wherein an inner and an outer surface of the extractor is a hydrophobic or super-hydrophobic surface.

Surfaces of components that come in contact with aerosol or residues of aerosol or of heated aerosol-forming substrate during a smoking process may be contaminated with these substances.

With the provision of hydrophobic, preferably super-hydrophobic, surfaces, formation of residues and condensation on these surfaces may be prevented or reduced. Cleaning of these parts of the aerosol-generating device may accordingly be omitted or reduced. In addition, cleaning is facilitated since contaminants from hydrophobic or super-hydrophobic surfaces in general come off easily.

In particular in electronic smoking devices comprising a heater blade and an extractor part where a tobacco containing aerosol-forming substrate is inserted into, cleaning may become a challenge, for example due to the fragility or limited accessibility of the elements.

Absence or a reduced presence of residues and by-products of the heating and aerosol-formation process in, for example, a flow conduit at or downstream of a heating region may also lead to an improved smoking experience and a more reliable and repeatable smoking experience. With a contaminant-free heater that is in direct contact with aerosol or aerosol-forming substrate the performance of the heater or of a given operation mode of the heater may be maintained.

Flow conduit is herein understood to include any surfaces in the aerosol-generating device that come into contact with substances of the heated aerosol-forming substrate. Thus, it includes any surface inside the device coming into contact with aerosol or with the heated aerosol-forming substrate and is typically arranged at and downstream of the heating region. The flow conduit may in particular include but is not limited to: a heating region or parts of the heating region; a flow conduit in a mouthpiece; surfaces defining a cavity for receiving an aerosol-forming substrate, for example a tobacco plug of an aerosol-generating article; an outer cartridge surface, which cartridge contains aerosol-forming substrate, which cartridge may for example be an integral part of the device or a non-replaceable or also a refillable cartridge.

A hydrophobic or super-hydrophobic surface may be a hydrophobic or super-hydrophobic coating applied to at least portions of inner surfaces of the main body in the heating region or downstream of the heating region. For example, a hydrophobic or super-hydrophobic coating may be applied to an inner surface of an air flow conduit, to at least portions of an outer surface of a heater or to inner surfaces defining a cavity in the main body for receiving aerosol-forming substrate, for example a heat stick or a tobacco plug. A hydrophobic or super-hydrophobic coating may be applied to surfaces of parts of an existing device, for example, to improve its cleaning properties.

A hydrophobic coating may, for example, be non-polar comprising by way of example silane, fluorocarbon, fluorinated compounds or acrylic acid. A non-polar or apolar coating or surface is defined by the absence of any localized electrical load.

A super-hydrophobic coating may, for example, comprise manganese oxide polystyrene nanocomposite, zinc oxide polystyrene nanocomposite, precipitate calcium carbonate, carbon nanotubes or may be a silica nanocoating.

Advantageously, hydrophobic coatings are selected in view of stability of the coating to insure that no degradation of the coating in function of the temperature and no chemical reaction with for example tobacco, nicotine based liquid and the aerosol generated in the device occurs.

Coatings may be applied to an underlying base material by methods known in the art for deposition of thin films. Chemical or physical deposition methods may be used. For example, a hydrophobic material may directly be sprayed on a surface of a material to be coated or dip coating of the material to be coated may be performed. More durable surface treatments are, for example, physical vapour deposition (PVD), chemical vapour deposition (CVD), sol-gel processes and other deposition processes suitable for thin-film coating.

A hydrophobic or super-hydrophobic surface may also be a micro-structured or nano-structured surface.

A super-hydrophobic surface may be a combination of a micro-structured and nano-structured surface. For example, micro structures of a micro-structured surface may be provided with nano-structures.

Micro-structured surfaces may have structure sizes in a range between 1 micrometer and several hundred micrometer, preferably between 1 and 200 micrometer, more preferably between 1 and 100 micrometer.

Nano-structured surfaces may have structure sizes of a few tenth or a few hundred nanometer. Nanostructures of nano-structured surfaces have sizes of, for example, between 10 nm and 800 nm, preferably between 50 nm and 500 nm, more preferably between 100 nm and 300 nm.

A hydrophobic or super-hydrophobic coating may be applied to a micro-structured or nano-structured surface or to a micro-structured and nano-structured surface.

A hydrophobic or super-hydrophobic coating applied to a micro- or nano-structured surface may be any one of the hydrophobic or super-hydrophobic coatings mentioned above.

A micro- or nanostructure may be provided to a hydrophobic or super-hydrophobic coating.

By a combination of structure and coating, physical properties of the structured surface may be combined with chemical properties of the coating. A hydrophobic or super-hydrophobic effect may thus be enhanced or adapted to specific substances to be repelled from the respective surfaces. For example, a surface may be adapted to be super-hydrophobic for a specific aerosol droplet size or specific viscosity of an aerosol-forming liquid to be aerosolized.

Chemical properties of coatings may be enhanced when a micro- or nanostructure of the surface is created. This may become significant when a micro- or nano-structuration created on a surface is related to a droplet size. For example, a super-hydrophobic surface may be achieved by dispersing artificial asperities of micrometric sizes on a hydrophobic surface. Ultra-hydrophobicity is reached, when a liquid droplet rests on the top of such asperities.

Hydrophobicity or hydrophilicity of a surface is defined by the contact angle θ between a droplet and a flat solid surface the droplet is arranged on. The contact angle is a measure of the wettability of the surface by the droplet.

A hydrophobic surface has a contact angle θ larger than 90 degree. A contact angle θ of hydrophobic surfaces is typically between 90 degree and 120 degree (a droplet beads up). A contact angle of about 90 degree is an ideal contact angle for water on a surface, indicating that the surface is water-repellent, but will still clean with water.

Super-hydrophobic surfaces are characterized by a large contact angle and are difficult to wet with water.

A super-hydrophobic surface has a contact angle of larger than 150 degree. A contact angle θ of super-hydrophobic surfaces are typically between 150 degree and 180 degree (a droplet is highly beaded)

Contrary to hydrophobicity, on a hydrophilic surface a water droplet spreads out far and the contact angle θ is very small. On these surfaces, the water droplets do not roll, but glide.

As used herein, the term 'droplet size' is used to mean the aerodynamic droplet size, which is the size of a spherical unit density droplet that settles with the same velocity as the droplet in question. Several measures are used in the art to describe aerosol droplet size. These include mass median diameter (MMD) and mass median aerodynamic diameter (MMAD). As used herein, the term 'mass median diameter (MMD)' is used to mean the diameter of a droplet such that half the mass of the aerosol is contained in small diameter droplets and half in large diameter droplets. As used herein, the term 'mass median aerodynamic diameter (MMAD)' is used to mean the diameter of a sphere of unit density that has the same aerodynamic properties as a droplet of median mass from the aerosol.

The mass median aerodynamic diameter (MMAD) of the droplets generated by the smoking device of the present invention may be between about 0.1 µm and about 10 µm, or the MMAD may be between about 0.5 µm and about 5 µm, for example between 0.5 µm and 3 µm such as between 0.8 µm and 1.2 µm. The MMAD of the droplets may be equal to or less than 2.5 µm. The desired droplet size of the droplets generated by the smoking device of the present invention may be any MMAD described above. The desired droplet size (MMAD) may be equal to or less than 2.5 µm.

Preferably, an aerosol-forming liquid used for aerosol generation in the smoking device according to the invention has a viscosity in a range between 1 mPas and 200 mPas, preferably between 1 mPas and 150 mPas, for example between 80 mPas and 130 mPas.

Micro-structured or nano-structured surfaces may be manufactured by methods known in the art for manufacturing nano-structured and micro-structured surfaces. Micro-structured or nano-structured surfaces may, for example, be manufactured by micro-shot peening, femtosecond laser machining, microplasma treatment, photolithography or nanoimprinting lithography.

Micro-shot peening is a cold working process modifying the mechanical properties of a surface, for example a metal or composite surface. In micro-shot peening, a surface is impacted with shot (round particles, for example made of glass, metal of ceramic material) sufficient to create plastic deformation. Thereby, each particle functions as a ball-peen hammer such that the surface is plastically deformed (contrary to sandblasting where abrasion takes place). Micro-shot peening is favourable in manufacturing random surface structures, for example, providing a surface with micro-dimples. Compared to conventional shot-peening in micro-shot peening smaller shot dimensions and higher shot velocity is used. Desired structures on a given surface material and geometry may be achieved by appropriate selection of the shot material and size, shot intensity and coverage. For micro-shot peening typical shot sizes of between 0.03 mm to 0.5 mm are used. For surface treatment of surfaces in smoking devices, preferably, shot comprising of particles having sizes of about 0.03 mm in diameter are used.

With femtosecond laser micro- and nanostructures may be performed in one step. This method is basically suitable for all materials and is easily scalable to a desired structure size and structure pattern to be achieved. Random structures are available as well as periodic structures. In particular nanostructures of a few tenth or a few hundred nanometer, for example, 100 nm to 300 nm, or down to 10 nm are available with femtosecond laser treatment. The method is contactless and does not need to be but may be performed in special atmospheres. Microstructures by femtosecond laser treatment are preferably in a lower micrometer range, typically in the range of 1 micrometer to 20 micrometer.

Microstructures may be provided with sub-micron and smaller microstructures or nanostructures, respectively.

Microplasma treatment compared to conventional plasma treatment has the advantage of being limited in dimensions of the plasma, ranging from tens to thousands of micrometer. Plasma treatment of a surface not only allows to deposit or etch a material but also to activate or chemically alter a surface. Thus, microplasma treatment allows to provide a structure to a surface and hydrophobization of specific materials may be achieved. For example hydrophobic fluorocarbon polymers pattern may be provided on a glass substrate.

Photolithography is an as such well known process, for example, in wafer structuration and will not further be described. Nanoimprinting lithography (NIL) is basically a similar process. A resist on a substrate is provided with a desired structure by a structured stamp. The structured resist may be etched and cured to achieve the final structure. Instead of a resist a molding material may directly be provided with a structure of a stamp. Due to the nature of polymer-to-polymer contact printing in lithography, the elastic constitution of the material allows for the manufacture of microstructures down to about 1 micrometer. However, depending on the material, for example amorphous metal, nanostructures down to about 20 to 100 nanometer may be provided.

Advantageously, a hydrophobic or super-hydrophobic surface in the device according to the invention, in particular surfaces of an airflow conduit, comprise a hydrophobic or super-hydrophobic surface optimized for the above-mentioned aerosol droplet sizes.

Depending on the set-up of the device and the way the aerosol-forming substrate is provided, for example using a liquid aerosol-forming reservoir or a solid tobacco material, different parts of the device or of the flow conduit come into contact with heated aerosol-forming substrate, aerosol or other evaporated or non-evaporated substances from the aerosol-forming substrate. Thus, different parts and elements of these devices may be provided with a hydrophobic or super-hydrophobic surface.

A device may comprise a compartment for holding aerosol-forming substrate. Such a compartment may directly receive solid aerosol-forming substrate or may receive or contain a cartridge or reservoir. If in direct contact with aerosol-forming substrate or possibly forming part of an airflow conduit guiding evaporated substances to a mouth end of the device, the inner compartment walls are preferably hydrophobic or super-hydrophobic surfaces.

For example, in devices for use together with a heat stick, which typically comprises an aerosol-forming substrate containing tobacco plug, the main body comprises a cavity for receiving an aerosol-forming substrate to be heated in the cavity or possibly also for receiving a cartridge to be inserted into the cavity. Cavities in main bodies for receiving an aerosol-generating substrate are typically in direct contact with the substrate, in particular with the heated substrate and as such preferably comprise a hydrophobic or super-hydrophobic inner surface defining the cavity. Such cavities may more easily be cleaned before a new aerosol-forming substrate is inserted into the cavity.

A device for being used with a heat stick further comprises a heater arranged in the main body, which heater is adapted for heating aerosol-forming substrate accommodated in the cavity. Preferably, in these devices, at least portions of surfaces defining the cavity or of the heater are a hydrophobic or super-hydrophobic surface.

In resistively heatable devices, the heater may be a heater blade extending into the cavity. A heat stick may be introduced with its aerosol-forming substrate containing end or tobacco end, into the cavity of the device and is thereby pushed over the heater blade. To support consistent heating and smoking conditions for each new heat stick, the heater blade may comprise a hydrophobic or super-hydrophobic outer surface.

The cavity or main body device walls in the heating region surrounding the cavity may be formed by one or preferably several elements. These elements not only define the cavity for the aerosol-forming substrate but may also define an air flow path inside the main body from the environment to the heated aerosol-forming substrate. The inside of the cavity and such an external air flow path may be in fluid connection with each other such that evaporated substances might pass from the cavity into the external air flow path and be deposited therein.

The heating region comprises a tubular-shaped extractor, wherein an inner surface and an outer surface of the extractor is a hydrophobic or super-hydrophobic surface. The inner surface of the extractor thereby substantially defines the size of the cavity.

In devices where aerosol-forming substrate is provided in liquid form or liquid containing form, such as for example in a cartridge or a tank system, the set-up of the device is different and a user draws on a mouthpiece of the device.

In such embodiments of electronic smoking devices, the main body comprises a proximal end and comprises an air flow conduit leading from the heating region to the proximal end of the main body inside the main body. At least portions of the air flow conduit may comprise a hydrophobic or super-hydrophobic surface. A heater for heating the aerosol-forming substrate may be part of the device and accordingly also comprise a hydrophobic or super-hydrophobic surface. However, the heater may also be integral with, for example, a cartridge. Preferably, in systems where a cartridge is reusable, comprising a heater or not, an outside of the cartridge may comprise a hydrophobic or super-hydrophobic surface.

The main body of the device may also comprise a reservoir for holding an aerosol-forming substrate. An outer surface of such a reservoir or cartridge may be provided with a hydrophobic or super-hydrophobic surface. This measure is in particular favourable if the reservoir is an integrated reservoir or reusable reservoir or also generally a refillable reservoir in the form of a cartridge or tank. Although reusable cartridges are often individual and removable parts independent of the device, with hydrophobic outer surfaces, cleaning of these cartridges before reuse may be omitted or simplified.

Electronic smoking devices may comprise an aerosolization chamber where substances evaporated from the aerosol-forming substrate may, for example, cool down and form an aerosol. Typically, such an aerosolization chamber is arranged downstream of the heating region and typically also downstream of a heater, basically independent on the way of heating the aerosol-forming substrate or in what state and form the substrate is provided.

In embodiments of the device according to the invention comprising an aerosolization chamber arranged downstream of the heating region, for example where a flow conduit comprises an aerosolization chamber, the aerosolization chamber preferably comprises a hydrophobic or super-hydrophobic inner surface.

Heaters may be in direct contact with aerosol-forming substrate or may be mounted, for example, in a device housing thus protected by housing walls. Heaters may be a part of the device or may be part of a cartridge. Preferably, heaters are resistive or inductive heaters.

In devices comprising a heater, which is in direct contact with aerosol-forming substrate, the surface of the heater is preferably provided with a hydrophobic or super-hydrophobic surface, in particular if the heater is not a replaceable heater such as for example a heater which is part of a disposable cartridge. In devices comprising a heater, for example in the form of a fluid permeable flat mesh heater or in the form of a heater blade, the mesh heater or the heater blade preferably comprises a hydrophobic or super-hydrophobic outer surface. For example, a glass blade and an aerosolization chamber made of glass may comprise hydrophobic or super-hydrophobic surfaces.

An aerosol-generating device may further comprise a mouthpiece, wherein a portion of the flow conduit, a most downstream portion of the flow conduit, is arranged in the mouthpiece.

'Upstream' and 'downstream' is herein seen in view of a flow direction of air entering the device, passing through the device and leaving the device. An outlet opening of the device is a most downstream location in a device where an air flow, comprising or not comprising aerosol, leaves the device.

The flow conduit of a device may be comprised of one or several individual, separate or intertwined flow channels.

The device according to the invention may comprise a mouthpiece comprising at least a flow channel arranged in the mouthpiece. A downstream end of the flow channel corresponds to an outlet opening in the proximal end of the mouthpiece. The flow channel in the mouthpiece may comprise a hydrophobic or super-hydrophobic surface.

The invention is further described with regard to embodiments, which are illustrated by means of the following drawings, wherein:
- Figs. 1, 2: show hydrophobicity and hydrophilicity of a surface;
- Fig. 3: shows a hydrophobic nano- and microstructured surface;
- Fig. 4: is a schematic cross section of an electronic smoking device using a heater blade for heating a tobacco plug of an aerosol-generating article;
- Fig. 5: shows an electronic smoking device comprising a liquid containing reservoir.

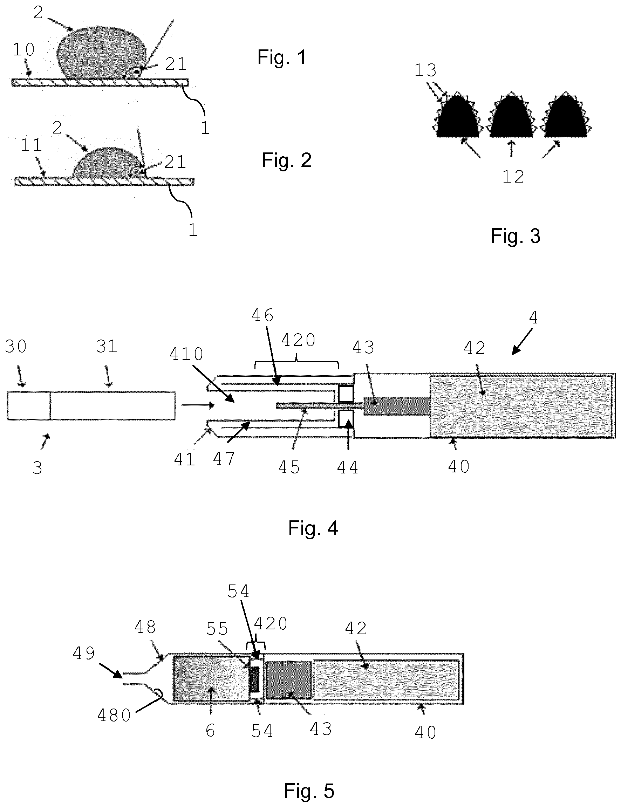

**Fig. 1** and **Fig. 2** show examples of a droplet behaviour on a hydrophobic and a hydrophilic surface. The main method for distinguishing between hydrophobic and hydrophilic surfaces 10,11 is the contact angle 21. This refers to the angle 21 that a droplet of fluid, here an aerosol droplet 2, makes at the point of contact with the surface of a rigid substrate 1. For a hydrophobic surface 10 as shown in Fig. 1, the contact angle 21 is always larger than 90 degree, and it can be as high as 150 degree. At contact angles above 150 degree a surface is called super-hydrophobic. Hydrophilic surfaces 11 as shown in Fig. 2 always have contact angles less than 90 degrees and usually less than 50 degree.

In **Fig. 3** an example of a combined micro- and nanostructure is shown. The microstructures 12 are in the form of domes 12, which may be regularly or irregularly arranged on a surface. The microstructures 12 itself are provided with nanostructures 13 shown as a plurality of pyramids on the surfaces of the microstructures 12. Super-hydrophobicity is available when an aerosol droplet 2 remains on top of the nanostructures 13.

**Fig. 4** is an example of an electronic smoking device 4 for heat sticks 3. An aerosol-generating article in the form of a tobacco stick comprises a mouth portion 30 and a tobacco portion 31. The mouth portion 30 comprises, for example, a filter segment. The tobacco portion 31 may comprise a tobacco plug, for example, a crimped and gathered cast leaf (a form of reconstituted tobacco that is formed from a slurry including tobacco particles, fiber particles, aerosol former, binder and for example also flavours). The heat stick 3 is inserted into a cavity 410 in a front housing portion 41 of the device 4. At least the mouth portion 30 of the heat stick 3 extends from the front housing portion when the stick 3 is accommodated in the cavity 410. In use, a user draws on the mouth portion 30 of the heat stick 3 for drawing aerosol and other substances from the heated tobacco stick through the stick to the mouth portion 30.

The front housing portion 41 is basically formed by an extractor 47 and a middle part 46, the latter for example being made of aluminium. A heater blade 45 for heating the tobacco extends into the cavity 410 and is pushed into the tobacco portion 31 of the heat stick 3 upon insertion of the heat stick 3 into the cavity 410 of the device 4. The region where the front housing portion 47 surrounds the heater blade and an aerosol-forming substrate when present in the cavity basically forms the heating region 420 of the device. In a radial extension, the heating region 420 extends up to the inner side of the front housing portion's 41 outer wall.

A distal housing portion 40 comprises a battery 42 and electronics, for example an electronic control board 43, connected to the heater blade 45 for heating the heater blade and controlling a heating process. A thermal insulation, for example a heater overmould 44, is arranged between cavity 410 and distal housing portion 40 for separating the heating region 420 from the distal housing portion 40. Heater overmould 44 directly forms the bottom of the cavity 410.

The parts of the front housing portion 41, that is, the extractor 47 and the middle part 46, as well as the heater blade and the heater overmould 44 are provided with a hydrophobic or preferably with a super-hydrophobic surface. These may be nanostructured surfaces, micro-structured surfaces, hydrophobic or super-hydrophobic coatings or a combination thereof.

Preferably, extractor 47 and middle part 46 are provided with a hydrophobic surface on all sides, at least on an inner and outer side. The heater overmold 44 may be provided with a hydrophobic surface only on that side forming part of the cavity 410. The heater blade 45 is provided with a hydrophobic surface at least on the two large sides, preferably also on the small sides.

Extractor 47 and middle part 46 have a basically tubular shape. However, an extractor 47 is typically provided with several protrusions and indentations and a middle part is provided with several openings for an airflow to be able to pass through. Thus, these parts are either in direct contact with the tobacco portion or may come in contact with substances from the heated tobacco substrate or with both and are thus prone to capture residues on their surfaces. In addition, their shape and fragility provide limited accessibility and stability in view of cleaning. Since the smoking device 4 is made for repeated use, reducing accumulation of contaminants and residues on the parts coming in direct contact with substances from the heated tobacco substrate of the heat stick 3, handling, performance and a user experience may be enhanced by the provision of hydrophobic surfaces of these parts of the device.

In **Fig. 5** an example of an electronic smoking device using a liquid reservoir or tank 6 is shown. The liquid aerosol-forming substrate in the tank 6 is heated and aerosolized by a heater 55, for example a heater coil around a wick element or a fluid permeable flat heater. The heater 55 is arranged adjacent to the tank 6. Laterally next to the heater 55, electrical contacts 54 are provided to supply power from the battery 42 to the heater 55. The battery 42 and electronics, for example an electronic control board 43 are arranged in a distal housing portion 40.

The heating region 420 in the embodiment shown in Fig. 5 has a rather small extension in a longitudinal direction of the device and is in this longitudinal direction basically limited to the region between open end of the tank 6 and the size of the heater. In a radial extension the heating region 420 extends up to the inner side wall of the main body.

The proximal end of the device and a most downstream element of the device housing is formed by a mouthpiece 48. In the mouthpiece 48 the aerosol from the heated liquid is collected and may leave the mouthpiece 48 via outlet 49. In these devices a user draws on the mouthpiece of the device.

Aerosol generated at the location of the heater 55 passes between tank 6 and housing wall downstream to the mouthpiece 48 and to the outlet 49. Thus, surfaces of parts and elements on this path are preferably all hydrophobic surfaces or super-hydrophobic surfaces.

The heater 55, the internal housing wall arranged next to the tank 6, the internal surfaces 480 of the mouthpiece 48 as well as the outer surface of the tank 6 are hydrophobic or super-hydrophobic surfaces.

In the embodiment shown in Fig. 5 the mouthpiece comprises a cavity forming an aerosolization chamber arranged downstream of the tank 6.

The tank 6 may be an integrated tank or may be a replaceable and possibly refillable tank 6.

It will be understood that mentioning the provision of a hydrophobic surface also includes the possibility of providing a super-hydrophobic surface. In view of reduced contaminations and simplified cleaning properties super-hydrophobic surfaces may be preferred. However, depending on a material used or for example cost constraints, different processes for making a surface hydrophobic or super-hydrophobic may be chosen.

## Claims

1. Electronic aerosol-generating smoking device (4) comprising a main body comprising:
a cavity (410) for receiving an aerosol-forming substrate;
a heater (55) arranged in the main body and adapted for heating aerosol-forming substrate accommodated in the cavity (410); and
a heating region (420) for aerosol-forming substrate, the heating region (420) comprising a tubular-shaped extractor, wherein an inner and an outer surface of the extractor is a hydrophobic or super-hydrophobic surface;
wherein at least a portion of an internal surface (480) of the main body defining the cavity (410) in the heating region (420) is a hydrophobic or super-hydrophobic surface.

2. Device (4) according to claim 1, wherein the heater (55) is a heater blade (45) extending into the cavity (410), the heater blade (45) comprising a hydrophobic or super-hydrophobic outer surface.

3. Device (4) according to claim 1, wherein the main body comprises a proximal end, and
an air flow conduit leading from the heating region (420) to the proximal end of the main body, wherein at least portions of the air flow conduit comprises a hydrophobic or super-hydrophobic surface.

4. Device (4) according to claim 1 or 3, wherein the air flow conduit comprises an aerosolization chamber arranged downstream of the heating region, the aerosolization chamber comprising a hydrophobic or super-hydrophobic inner surface.

5. Device (4) according to any one of claims 1, 3 or 4, comprising a mouthpiece (48) comprising a flow channel arranged in the mouthpiece (48), wherein a downstream end of the flow channel is an outlet opening in the proximal end of the mouthpiece (48), the flow channel in the mouthpiece (48) comprising a hydrophobic or super-hydrophobic surface.

6. Device (4) according to any one of claims 1, 3 to 5, the main body comprising a reservoir (6) for holding an aerosol-forming substrate, wherein an outer surface of the reservoir is provided with a hydrophobic or super-hydrophobic surface.

7. Device (4) according to any one of the preceding claims, wherein the hydrophobic or super-hydrophobic surface is a hydrophobic or super-hydrophobic coating applied to at least the portions of inner surfaces of the main body in the heating region (420) or downstream of the heating region (420).

8. Device (4) according to claim 7, where the hydrophobic coating is non-polar comprising silane, fluorocarbon, fluorinated compounds or acrylic acid.

9. Device (4) according to claim 7, where the super-hydrophobic coating comprises manganese oxide polystyrene nanocomposite, zinc oxide polystyrene nanocomposite, precipitate calcium carbonate, carbon nanotubes or is a silica nanocoating.

10. Device (4) according to any one of claims 1 to 6, wherein the hydrophobic or super-hydrophobic surface is a micro-structured or nano-structured surface.

11. Device (4) according to claim 10, wherein the super-hydrophobic surface is a combination of a micro-structured and nano-structured surface.

12. Device (4) according to any one of claims 10 or 11, wherein the micro-structured or nano-structured surface is manufactured by micro-shot peening, femtosecond laser machining, microplasma treatment, photolithography or nanoimprinting lithography.

13. Device (4) according to any one of claims 10 to 12, wherein a hydrophobic or super-hydrophobic coating is applied to a micro-structured or nano-structured surface.

## Patentansprüche

1. Elektronische aerosolerzeugende Vorrichtung zum Rauchen (4), die einen Hauptkörper aufweist, aufweisend:
einen Hohlraum (410) zum Aufnehmen eines aerosolbildenden Substrats;
eine Heizvorrichtung (55), die im Hauptkörper angeordnet und zum Erwärmen eines aerosolbildenden Substrats, das im Hohlraum (410) aufgenommen ist, angepasst ist; und
einen Heizbereich (420) für ein aerosolbildendes Substrat, wobei der Heizbereich (420) einen röhrenförmigen Extraktor aufweist, wobei eine Innen- und eine Außenfläche des Extraktors eine hydrophobe oder superhydrophobe Fläche sind;
wobei zumindest ein Teil einer Innenfläche (480) des Hauptkörpers, der den Hohlraum (410) in dem Heizbereich (420) definiert, eine hydrophobe oder superhydrophobe Fläche ist.

2. Vorrichtung (4) nach Anspruch 1, wobei die Heizvorrichtung (55) eine Heizklinge (45) ist, die sich in den Hohlraum (410) erstreckt, wobei die Heizklinge (45) eine hydrophobe oder superhydrophobe Außenfläche aufweist.

3. Vorrichtung (4) nach Anspruch 1, wobei der Hauptkörper ein proximales Ende aufweist, und
eine Luftströmungsleitung, die von dem Heizbereich (420) zu dem proximalen Ende des Hauptkörpers führt, wobei zumindest Teile der Luftströmungsleitung eine hydrophobe oder superhydrophobe Fläche aufweisen.

4. Vorrichtung (4) nach Anspruch 1 oder 3, wobei die Luftströmungsleitung eine Aerosolisierungskammer aufweist, die nachgeschaltet des Heizbereichs angeordnet ist, wobei die Aerosolisierungskammer eine hydrophobe oder superhydrophobe Innenfläche aufweist.

5. Vorrichtung (4) nach einem der Ansprüche 1, 3 oder 4, aufweisend ein Mundstück (48), das einen Strömungskanal aufweist, der in dem Mundstück (48) angeordnet ist, wobei ein nachgeschaltetes Ende des Strömungskanals eine Auslassöffnung in dem proximalen Ende des Mundstücks (48) ist, wobei der Strömungskanal in dem Mundstück (48) eine hydrophobe oder superhydrophobe Fläche aufweist.

6. Vorrichtung (4) nach einem der Ansprüche 1, 3 bis 5, wobei der Hauptkörper einen Vorratsbehälter (6) zum Halten eines aerosolbildenden Substrats aufweist, wobei eine Außenfläche des Vorratsbehälters mit einer hydrophoben oder superhydrophoben Fläche vorgesehen ist.

7. Vorrichtung (4) nach einem der vorhergehenden Ansprüche, wobei die hydrophobe oder superhydrophobe Fläche eine hydrophobe oder superhydrophobe Beschichtung ist, die auf zumindest die Abschnitte der Innenflächen des Hauptkörpers in dem Heizbereich (420) oder nachgeschaltet des Heizbereichs (420) aufgebracht ist.

8. Vorrichtung (4) nach Anspruch 7, wobei die hydrophobe Beschichtung unpolar ist, aufweisend Silan, Fluorkohlenstoff, fluorierte Verbindungen oder Acrylsäure.

9. Vorrichtung (4) nach Anspruch 7, wobei die superhydrophobe Beschichtung Manganoxid-Polystyrol-Nano-Verbundwerkstoff, Zinkoxid-Polystyrol-Nano-Verbundwerkstoff, ausgefallenes Calciumcarbonat, Kohlenstoff-Nanoröhren aufweist oder eine Siliciumdioxid-Nanobeschichtung ist.

10. Vorrichtung (4) nach einem der Ansprüche 1 bis 6, wobei die hydrophobe oder superhydrophobe Fläche eine mikrostrukturierte oder nanostrukturierte Fläche ist.

11. Vorrichtung (4) nach Anspruch 10, wobei die superhydrophobe Fläche eine Kombination einer mikrostrukturierten und einer nanostrukturierten Fläche ist.

12. Vorrichtung (4) nach einem der Ansprüche 10 oder 11, wobei die mikrostrukturierte oder nanostrukturierte Fläche durch Mikro-Kugelstrahlen, Femtosekundenlaserbearbeitung, Mikroplasmabehandlung, Fotolithografie oder Nanoimprinting-Lithografie hergestellt ist.

13. Vorrichtung (4) nach einem der Ansprüche 10 bis 12, wobei eine hydrophobe oder superhydrophobe Beschichtung auf eine mikrostrukturierte oder nanostrukturierte Fläche aufgebracht ist.

## Revendications

1. Dispositif à fumer de génération d'aérosol électronique (4) comprenant un corps principal comprenant :
une cavité (410) pour recevoir un substrat formant aérosol ;
un dispositif de chauffage (55) disposé dans le corps principal et adapté pour chauffer un substrat formant aérosol logé dans la cavité (410) ; et
une zone de chauffage (420) pour un substrat formant aérosol, la zone de chauffage (420) comprenant un extracteur de forme tubulaire, dans lequel une surface intérieure et une surface extérieure de l'extracteur sont une surface hydrophobe ou super-hydrophobe ;
dans lequel au moins une partie d'une surface intérieure (480) du corps principal définissant la cavité (410) dans la zone de chauffage (420) est une surface hydrophobe ou super-hydrophobe.

2. Dispositif (4) selon la revendication 1, dans lequel le dispositif de chauffage (55) est une lame chauffante (45) s'étendant dans la cavité (410), la lame chauffante (45) comprenant une surface extérieure hydrophobe ou super-hydrophobe.

3. Dispositif (4) selon la revendication 1, dans lequel le corps principal comprend une extrémité proximale, et
un conduit d'écoulement d'air conduisant de la zone de chauffage (420) à l'extrémité proximale du corps principal, dans lequel au moins des parties du conduit d'écoulement d'air comprennent une surface hydrophobe ou super-hydrophobe.

4. Dispositif (4) selon la revendication 1 ou 3, dans lequel le conduit d'écoulement d'air comprend une chambre d'aérosolisation disposée en aval de la zone de chauffage, la chambre d'aérosolisation comprenant une surface intérieure hydrophobe ou super-hydrophobe.

5. Dispositif (4) selon l'une quelconque des revendications 1, 3 ou 4, comprenant un embout buccal (48) comprenant un canal d'écoulement disposé dans l'embout buccal (48), dans lequel une extrémité aval du canal d'écoulement est une ouverture de sortie dans l'extrémité proximale de l'embout buccal (48), le canal d'écoulement dans l'embout buccal (48) comprenant une surface hydrophobe ou super-hydrophobe.

6. Dispositif (4) selon l'une quelconque des revendications 1, 3 à 5, le corps principal comprenant un réservoir (6) pour contenir un substrat formant aérosol, dans lequel une surface extérieure du réservoir est fournie avec une surface hydrophobe ou super-hydrophobe.

7. Dispositif (4) selon l'une quelconque des revendications précédentes, dans lequel la surface hydrophobe ou super-hydrophobe est un revêtement hydrophobe ou super-hydrophobe appliqué sur au moins les parties des surfaces intérieures du corps principal dans la zone de chauffage (420) ou en aval de la zone de chauffage (420).

8. Dispositif (4) selon la revendication 7, dans lequel le revêtement hydrophobe est non polaire comprenant du silane, du fluorocarbone, des composés fluorés ou de l'acide acrylique.

9. Dispositif (4) selon la revendication 7, dans lequel le revêtement super-hydrophobe comprend un nanocomposite de polystyrène d'oxyde de manganèse, un nanocomposite de polystyrène d'oxyde de zinc, du carbonate de calcium précipité, des nanotubes de carbone ou est un nanorevêtement de silice.

10. Dispositif (4) selon l'une quelconque des revendications 1 à 6, dans lequel la surface hydrophobe ou super-hydrophobe est une surface microstructurée ou nanostructurée.

11. Dispositif (4) selon la revendication 10, dans lequel la surface super-hydrophobe est une combinaison de surface microstructurée et nanostructurée.

12. Dispositif (4) selon l'une quelconque des revendications 10 ou 11, dans lequel la surface microstructurée ou nanostructurée est fabriquée par microgrenaillage, usinage au laser femtoseconde, traitement au microplasma, photolithographie ou lithographie par nano-impression.

13. Dispositif (4) selon l'une quelconque des revendications 10 à 12, dans lequel un revêtement hydrophobe ou super-hydrophobe est appliqué à une surface microstructurée ou nanostructurée.
